# DEMANDE DE BREVET EUROPEEN

(11) **EP 1 870 041 A2**
(43) Date de publication de la demande: **26.12.2007**
(21) Numéro de dépôt: 07110801.3
(22) Date de dépôt: 21.06.2007
(51) Int. Cl.: A61B 17/04, A61B 17/00, A61B 17/88

(54) **Elément d'ancrage de fil de suture**

(30) Priorité: 22.06.2006 FR 0605607
(71) Demandeur: Textile Hi-Tec (T.H.T.), 34220 Verreries de Moussans (FR)
(72) Inventeur: Houard, William, 81270, LABASTIDE-ROUAIROUX (FR)
(74) Mandataire: Hennion, Jean-Claude

(57) **Abrégé**

L'élément d'ancrage (1) de fil de suture est formé d'un corps monobloc fileté extérieurement présentant une partie distale, une face d'extrémité proximale, et deux évidements longitudinaux, traversant ledit corps et débouchant dans la face d'extrémité proximale et dans la partie distale (6), laquelle est de forme conique, avec une pointe d'extrémité (3) selon l'axe longitudinal (AA') de l'élément (1). Les deux évidements longitudinaux (7,7') débouchent, de façon excentrée par rapport à l'extrémité en pointe (3). La partie distale conique (6) comporte une rainure de liaison (11), reliant les deux évidements longitudinaux (7,7'), qui a une profondeur supérieure au diamètre du fil de suture.

De préférence l'élément comporte deux jeux (8,10) de deux évidements longitudinaux (7,7', 9,9') reliés par une rainure de liaison (11), formée dans la partie distale conique (6).

## Description

La présente invention concerne le domaine chirurgical, plus particulièrement des outils qui sont mis à la disposition du praticien pour réaliser l'ancrage d'un fil de suture dans une partie osseuse.

Dans le document US.5.370.662, l'élément d'ancrage se présente sous la forme d'une vis globalement cylindrique, avec une partie distale en pointe et un filetage périphérique. Cette vis comporte un prolongement proximal, percé d'un trou pour le passage d'un ou plusieurs fils de suture. Le vissage dans la partie osseuse se fait grâce à un outil d'insertion dont l'extrémité est configurée pour se bloquer sur l'arrière de la vis. Ce blocage peut être obtenu par exemple grâce à des encoches formées sur l'arrière du corps cylindrique et donc dans le filetage. Il peut également être obtenu grâce à des configurations particulières du prolongement proximal dans lequel est formé le trou de passage du ou des fils de suture. Il peut encore être obtenu en prolongeant le corps cylindrique par une portion spécialement dédiée à la coopération avec l'outil d'insertion.

Quel que soit le mode de réalisation, l'élément d'ancrage de ce document US.5.370.662 présente l'inconvénient de posséder le prolongement proximal, pour le passage du ou des fils de suture, qui dépasse de la partie osseuse et qui peut donc constituer une gêne.

De plus, quand bien même l'alésage réalisé dans la partie osseuse serait plus profond de manière à ce que le prolongement proximal pénètre dans ladite partie osseuse, il n'en reste pas moins vrai que cet élément d'ancrage présente une longueur totale qui est supérieure à la longueur réellement utile pour l'ancrage proprement dit, à savoir la longueur du filetage.

Cet inconvénient est en partie absent de l'élément d'ancrage décrit dans le document W0.96/28100, cet élément ne comportant pas de prolongement proximal percé d'un trou pour le passage du ou des fils de suture. Le corps cylindrique fileté sur sa périphérie n'est pas pointu mais présente une face d'extrémité distale circulaire. Cet élément comporte deux évidements longitudinaux internes qui débouchent dans chacune des deux faces d'extrémité proximale et distale. Le fil de suture passe d'un évidement longitudinal à l'autre avec une portion courbe venant en saillie au-delà de la face d'extrémité distale. Certes comme expliqué dans le document WO.96/28100, la réalisation de l'alésage dans la partie osseuse se fait à l'aide d'un foret pointu de sorte que la portion courbe du fil de suture en saillie ne vient normalement pas en contact avec la paroi osseuse au fond de l'alésage. Néanmoins, la présence de cette portion courbe en saillie du fil de suture peut être un inconvénient majeur si le praticien doit, à l'occasion du geste chirurgical, faire coulisser le fil notamment pour régler la longueur des deux brins. De plus dans ce document le blocage de l'élément d'ancrage avec l'outil d'insertion se fait par des découpes polygonales formées dans l'arrière du corps cylindrique, ce qui réduit d'autant l'efficacité réelle du filetage dans cette partie arrière.

Le but visé par la présente invention est de proposer un élément d'ancrage de fil de suture qui pallie tout ou partie des inconvénients précités.

Ce but est parfaitement atteint par un élément d'ancrage de fils de suture formé de manière connue par le document W0.86/28100 d'un corps monobloc fileté extérieurement, présentant une partie distale et une face d'extrémité proximale, ainsi que deux évidements longitudinaux, traversant ledit corps et débouchant d'une part dans la face d'extrémité proximale et d'autre part dans la partie distale. De manière caractéristique, la partie distale est de forme conique, avec une pointe d'extrémité selon l'axe longitudinal de l'élément, les deux évidements longitudinaux débouchant de façon excentrée par rapport à ladite extrémité en pointe. De plus sur la partie distale conique est formée une rainure de liaison, reliant les deux évidements longitudinaux, ladite rainure ayant une profondeur supérieure au diamètre du fil de suture.

Ainsi, selon la disposition particulière de la présente invention, l'élément d'ancrage comporte une tête de vissage pointue, ce qui améliore ses performances de vissage comparativement à celui qui est décrit dans le document W0.96/28100. De plus le fil de suture ne dépasse pas de l'extérieur de l'élément d'ancrage de sorte qu'il n'y a pas de risque de blocage lors du coulissement éventuel dudit fil de suture.

De préférence, l'élément d'ancrage selon la présente invention comporte deux jeux indépendants de deux évidements longitudinaux, chaque jeu étant relié par une rainure de liaison, formée dans la partie distale conique. L'élément peut donc servir d'ancrage à deux fils de suture indépendants l'un de l'autre, avec possibilité de faire coulisser un fil de suture donné sans interférer avec l'autre fil.

Selon ce mode préféré de réalisation, les deux jeux indépendants de deux évidements longitudinaux sont disposés symétriquement par rapport à un plan médian longitudinal de l'élément, ceci de manière à avoir un équilibre des forces de traction lors de la mise en tension des fils de suture. Ceci est notamment obtenu en disposant les quatre évidements longitudinaux, en section transversale, selon les quatre angles d'un carré ayant pour axe de symétrie l'axe longitudinal de l'élément.

Par ailleurs, on prévoit une empreinte permettant le placement de l'outil d'insertion. Cette empreinte est totalement indépendante des évidements de telle sorte qu'il n'y a aucun contact entre la tête de l'outil d'insertion avec les deux fils de suture enfilés dans l'ancre.

Selon une variante de réalisation, l'élément de suture est formé dans un matériau biodégradable ou résorbable, stérilisable, d'origine naturelle ou synthétique, notamment un matériau choisi dans le groupe :
- polylactide, polyglycolide, poly-ε-caprolactone,
- polyhydroxy butyrate, polyhydroxy valérate,
- poly carbonates et assimilés,
- cellulose, polysaccharides, amidon,
leurs homopolymères, leurs copolymères et leurs dérivés.

Ceci évite d'avoir à retirer l'élément d'ancrage, une fois que le fil de suture a rempli totalement la fonction qui lui était impartie ou de garder dans l'os ce corps étranger.

De préférence, dans ce cas, le matériau en question comporte une charge, sous la forme de poudre ou de nano poudre, à raison de 5 à 60 % en poids de charge, d'un composant choisi de préférence dans le groupe : hydroxy apatite et/ou de phosphate de calcium, notamment de β tricalcium phosphate, et/ou d'hydrogéno phosphate de calcium et/ou de carbonate de calcium et/ou d'hydrogéno carbonate de calcium.

La présence de ce type de charge permet de favoriser un développement homogène des cellules osseuses, venant se substituer à l'élément d'ancrage.

C'est un autre objet de la présente invention que de proposer un ensemble chirurgical comportant au moins un élément d'ancrage présentant les caractéristiques décrites ci-dessus et un outil d'insertion spécialement adapté pour réaliser le vissage dudit élément. De manière caractéristique, la face d'extrémité proximale de l'élément d'ancrage comporte une empreinte en creux pour le placement ajusté de la tête de l'outil d'insertion.

Lorsque l'élément d'ancrage comporte quatre évidements longitudinaux disposés, en section transversale, selon les quatre angles d'un carré, l'empreinte en creux est formée dans la partie centrale dudit carré, sans recoupement avec les quatre évidements longitudinaux.

De préférence, cette empreinte en creux a une configuration polygonale, notamment très légèrement conique. Il peut s'agir d'une configuration en étoile à quatre branches, l'extrémité de chacune desdites branches venant se loger dans l'espace entre deux évidements longitudinaux adjacents.

La présente invention sera mieux comprise à la lecture de la description qui va être faite d'un exemple préféré de réalisation d'un ensemble chirurgical pour la pose d'une ancre vissable pour deux fils de suture, illustré par le dessin annexé dans lequel :
La figure 1 est une vue schématique de côté de l'ancre vissable,
La figure 2 est une vue schématique de face depuis l'avant de l'ancre de la figure 1,
La figure 3 est une vue schématique de face depuis l'arrière de l'ancre de la figure 1,
La figure 4 est une vue schématique de côté de l'outil d'insertion
Et la figure 5 est une vue schématique de côté de l'outil d'insertion de la figure 4 sur lequel est positionnée l'ancre vissable.

L'ancre vissable 1 est destinée à être implantée dans une partie osseuse pour assurer la fixation d'au moins un fil de suture, ayant sa fonction propre liée au geste chirurgical auquel il est associé. L'implantation de l'ancre doit être suffisamment solide pour ne pas courir le risque d'une extraction de l'ancre de la partie osseuse lors de la mise en tension du fil de suture. Néanmoins la longueur de l'ancre est suffisamment réduite pour permettre son implantation dans les fragments osseux de petites dimensions.

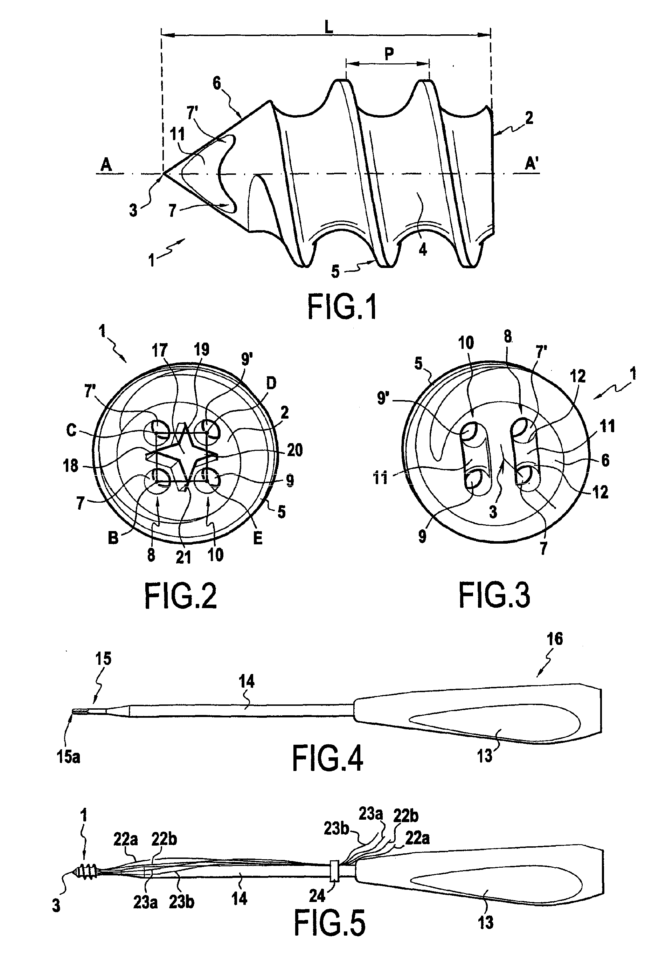

Dans le mode préféré de réalisation qui est illustré à la figure 1, l'ancre 1 a une longueur totale L, entre sa face proximale 2 et sa pointe distale 3 qui est de l'ordre de 10 à 12 mm.

L'ancre 1 est une pièce monobloc présentant un corps cylindrique 4 sur la périphérie duquel est prévu un filetage 5 s'étendant depuis la face proximale 2 jusqu'à la partie distale 6 de forme globalement conique terminée par la pointe distale 3, passant par l'axe longitudinal AA' de l'ancre 1.

L'ancre 1 est percée de deux jeux d'évidement longitudinaux, respectivement 7, 7' pour le premier jeu 8 et 9,9' pour le second jeu 10. Chaque évidement longitudinal 7,7', 9,9' est un trou cylindrique, parallèle à l'axe AA' , débouchant à la fois dans la face proximale 2 et dans la partie distale conique 6. Chaque jeu 8,10 sert au passage d'un seul fil de suture.

Au niveau de la partie distale conique d'extrémité 6, les deux évidements longitudinaux 7,7' du même jeu 8 sont reliés l'un à l'autre par une rainure 11 en creux dont la profondeur est supérieure au diamètre du fil de suture destiné à être utilisé avec ladite ancre 1. Cette disposition particulière vise à ce que le fil de suture, une fois enfilé dans les deux évidements longitudinaux 7,7' du même jeu 8, et mis sous tension depuis l'arrière de l'ancre 1, ledit fil ne dépasse pas de la surface extérieure de la partie distale conique 6. Le même fil de suture passe d'un premier évidement longitudinal 7 au deuxième évidement longitudinal 7' du même jeu 8 en formant une courbe qui pénètre dans la rainure de liaison 11, tandis que les deux brins libres dudit fil dépassent de la face proximale 2 et sont donc accessibles au praticien.

Dans l'exemple préféré, les deux jeux 8, 10, comportant chacun deux évidements longitudinaux respectivement 7,7' et 9,9', sont disposés symétriquement par rapport à un plan médian passant par l'axe longitudinal AA' de l'ancre 1. Plus précisément, chaque évidement longitudinal est centré sur les quatre coins d'un carré BCDE, lui-même centré sur l'axe longitudinal AA' de l'ancre (figure 3). Lors de la mise en tension des brins libres des deux fils de suture, il y a un parfait équilibrage des forces de traction mises en jeu, du fait en particulier de cette disposition parfaitement symétrique.

Comme cela apparaît à l'examen de la figure 2, le fond de la rainure 11, creusée dans la partie distale conique 6 en vue de relier l'un à l'autre les deux évidements longitudinaux 7, 7', est légèrement incurvé, formant un bord arrondi 12 de manière à éviter un coincement ou une usure du fil de suture lors de son coulissement à l'intérieur de l'ancre.

Dans l'exemple illustré, pour une longueur totale L faisant 10mm, le pas P du filetage est de 2,5mm, ledit filetage faisant globalement 2,5 spires de 360° entre la face d'extrémité proximale 2 et la partie distale conique 6. Le diamètre maximal de l'ancre, au niveau du filetage 5, est de l'ordre de 5,7 mm. Le diamètre de la face d'extrémité proximale 2 est de l'ordre de 3,5 mm. Chaque évidement longitudinal 7,7', 9,9' a un diamètre de l'ordre de 0,9 mm. Ce diamètre convient aux fils de suture les plus courants dans le domaine, faisant de l'ordre de 0,4 à 0,7 mm.

L'implantation de l'ancre 1 dans la partie osseuse est réalisée grâce à un outil d'insertion 16, assimilable à un tournevis spécialisé, avec un manche 13 et une tige 14 terminée par une tête 15 ayant une configuration adaptée pour venir se bloquer dans une empreinte 17 formée dans le corps cylindrique 4 à partir de la face d'extrémité proximale 2.

Dans l'exemple illustré à la figure 2, cette empreinte 17 a, en section transversale, la forme d'une étoile à quatre branches 18,19,20,21, axée sur l'axe AA' longitudinal de l'ancre 1. La configuration de cette empreinte, en creux dans l'ancre 1, se trouve à l'identique sur la tête 15 de l'outil d'insertion 16, mais en saillie de manière à ce que la tête 15 puisse venir se loger de manière ajustée dans l'empreinte 17. Pour faciliter l'introduction de l'extrémité 15a de la tête 15, l'empreinte 17 et la tête 15 ont un profil légèrement incliné vers l'avant, de sorte que l'extrémité 15a de la tête 15 est de dimension plus réduite que l'empreinte 17 au niveau de la face d'extrémité proximale 2.

La configuration en étoile à quatre branches pour l'empreinte 17 permet une très bonne localisation de ladite empreinte entre les quatre évidements longitudinaux 7,7', 9,9', comme cela apparaît à l'examen de la figure 3. Les quatre branches 18, 19, 20, 21 de l'étoile viennent se loger dans l'espace entre les deux évidements longitudinaux, respectivement 7,7' pour la première branche 18, 7',9' pour la deuxième branche 19, 9', 9 pour la troisième branche 20 et 9,7 pour la quatrième branche 21. L'empreinte 17 est donc totalement indépendante des évidements longitudinaux 7,7', 9,9' de telle sorte qu'il n'y a aucun contact entre la tête 15 de l'outil d'insertion 16 avec les deux fils de suture enfilés dans l'ancre 1.

La longueur de l'empreinte à l'intérieur du corps cylindrique 4 est suffisamment importante pour garantir le vissage sans problème de l'ancre 1 dans la partie osseuse. Dans l'exemple précis donné ci-dessus, à titre non exhaustif, cette longueur est de 5,5 mm, avec un angle d'inclinaison de l'ordre de 2°.

L'angle au sommet de la partie distale conique 6 est de l'ordre de 70°.

Sur la figure 5 on a représenté l'ancre 1 montée sur la tête 15 de l'outil d'insertion 16, avec les deux fils de suture 22, 23 dont les deux brins libres 22a, 22b, 23a, 23b sont réunis et bloqués contre la tige 14 de l'outil 17 grâce à une pièce 24 de blocage, venant s'emboîter sur ladite tige par-dessus les deux fils 22, 23. Cette pièce 24 n'empêche pas le libre coulissement d'un fil de suture le long de la tige 14 et à l'intérieur de l'ancre 1 lors de la mise en tension dudit fil de suture, notamment lorsque le praticien tire sur l'un des brins dudit fil.

On comprend que la tête 15 est particulièrement adaptée à une empreinte 17 donnée. L'ensemble chirurgical mis à la disposition du praticien peut comprendre plusieurs ancres 1 de tailles différentes suivant les applications, toutes comportant la même empreinte 17. Dans ce cas le praticien peut utiliser le même outil d'insertion 16 avec la même tige 14 et la même tête 15. Cependant il peut être préférable d'adapter l'empreinte 17 à la taille de l'ancre 1 et par conséquent de disposer d'un outil d'insertion 16 qui soit parfaitement adapté à chaque empreinte. Dans ce cas, de préférence, il y a autant de tiges 14 que de sortes d'empreintes, mais un seul manche 16 apte à coopérer, pour le vissage, avec l'extrémité proximale de toutes lesdites tiges 14.

L'ancre 1 peut être métallique, dans un matériau biocompatible conventionnel. Elle peut également être dans un matériau biodégradable ou résorbable, stérilisable, d'origine naturelle ou synthétique, notamment un matériau choisi dans le groupe suivant :
- polylactide, polyglycolide, poly-ε-caprolactone,
- polyhydroxy butyrate, polyhydroxy valérate,
- poly carbonates et assimilés,
- cellulose, polysaccharides, amidon,
leurs homopolymères, leurs copolymères et leurs dérivés.

De préférence, dans ce cas, le matériau biodégradable ou résorbable contient une charge, sous forme de poudre ou de nano poudre dans une proportion de 5 à 60%, ladite charge étant notamment choisie dans le groupe : hydroxy apatite et/ou de phosphate de calcium, notamment de β tricalcium phosphate, et/ou d'hydrogéno phosphate de calcium et/ou de carbonate de calcium et/ou d'hydrogéno carbonate de calcium, de préférence à raison de 5 à 60% en poids de charge.

Le choix du matériau biodégradable ou résorbable ainsi que le choix de la charge permet d'ajuster l'élimination de l'ancre et son remplacement par une partie osseuse régénérée dans une durée qu'il est possible de déterminer, notamment en terme d'évolution des propriétés mécanique. Par exemple les propriétés mécaniques , en terme de force de rupture de l'ancre, pourront évoluer avec une paire de l'ordre de 50% des propriétés initiales au bout de six mois et une perte totale des propriétés initiales au bout d'un an.

## Revendications

1. Elément d'ancrage (1) de fil de suture formé d'un corps monobloc fileté extérieurement présentant une partie distale, une face d'extrémité proximale (2), et deux évidements longitudinaux, traversant ledit corps et débouchant d'une part dans la face d'extrémité proximale et d'autre part dans la partie distale **caractérisé en ce que** :
- la partie distale (6) est de forme conique, avec une pointe d'extrémité (3) selon l'axe longitudinal (AA') de l'élément (1), et **en ce que**, les deux évidements longitudinaux débouchant, de façon excentrée par rapport à l'extrémité en pointe, la partie distale conique (6) comporte une rainure de liaison (11), reliant les deux évidements longitudinaux (7,7'), ayant une profondeur supérieure au diamètre du fil de suture ;
- l'élément d'ancrage comporte une empreinte en creux (17) pour le placement d'une tête (15) de l'outil d'insertion (16) indépendante des évidements longitudinaux (7,7').W

2. Elément d'ancrage selon la revendication 1 **caractérisée en ce qu'**elle comporte deux jeux (8,10) de deux évidements longitudinaux (7,7', 9,9') reliés par une rainure de liaison (11), formée dans la partie distale conique (6).

3. Elément d'ancrage selon la revendication 2 **caractérisée en ce que** les deux jeux (8,10) sont disposés symétriquement par rapport à un plan médian longitudinal de symétrie.

4. Elément d'ancrage selon la revendication 3 **caractérisée en ce que** les quatre évidements longitudinaux (7,7',9,9') sont disposés, en section transversale, selon les quatre angles (B,C,D,E) d'un carré ayant pour axe de symétrie l'axe longitudinal (AA') de l'élément.

5. Elément d'ancrage selon l'une des revendications 1 à 4 **caractérisée en ce qu'**il est formé dans un matériau résorbable, stérilisable, d'origine naturelle ou synthétique, notamment choisi dans le groupe :
- polylactide, polyglycolide, poly-ε-caprolactone,
- polyhydroxy butyrate, polyhydroxy valérate,
- poly carbonates et assimilés,
- cellulose, polysaccharides, amidon,
leurs homopolymères, leurs copolymères et leurs dérivés.

6. Elément d'ancrage selon la revendication 5 **caractérisée en ce que** le matériau est chargé de poudre et/ou de nano poudre, notamment d'un composant choisi dans le groupe : hydroxy apatite et/ou de phosphate de calcium, notamment de β tricalcium phosphate, et/ou d'hydrogéno phosphate de calcium et/ou de carbonate de calcium et/ou d'hydrogéno carbonate de calcium, de préférence à raison de 5 à 60% en poids de charge.

7. Ensemble chirurgical comportant au moins un élément d'ancrage (1) selon l'une des revendications 1 à 6 et un outil d'insertion (16) spécialement adapté pour réaliser le vissage dudit élément (1) **caractérisé en ce que** la face d'extrémité proximale (2) de l'élément d'ancrage (1) comporte une empreinte en creux (17) pour le placement ajusté de la tête (15) de l'outil d'insertion (16).

8. Ensemble selon la revendication 7 **caractérisé en ce que** l'empreinte (17) en creux a une configuration polygonale, notamment très légèrement inclinée vers l'avant de l'élément.

9. Ensemble selon la revendication 8 **caractérisé en ce que** l'empreinte (17) a une configuration en étoile à quatre branches, chacune desdites branches (18,19,20,21) venant se loger dans l'espace entre deux évidements longitudinaux adjacents.
